# EUROPEAN PATENT SPECIFICATION

(11) **EP 1 210 911 B1**
(45) Date of publication and mention of the grant of the patent: **19.01.2011**
(21) Application number: 01309716.7
(22) Date of filing: 19.11.2001
(51) Int. Cl.: A61B 10/00

(54) **Bone marrow biopsy needle**
Knochenmarkbiopsienadel
Aiguille pour biopsie de moelle osseuse

(30) Priority: 23.11.2000 GB 0028542
(43) Date of publication of application: 05.06.2002
(73) Proprietor: Islam, Anwarul, Amherst, NY 14226 (US)
(72) Inventor: Islam, Anwarul, Amherst, NY 14226 (US)
(74) Representative: Davies, Gregory Mark

(56) References cited:
- WO-A-82/03167
- WO-A-99/34734
- US-A- 4 314 565
- US-A- 4 403 617

## Description

The present invention relates to a biopsy needle for use in taking a bone marrow biopsy sample from, for example, posterior iliac crests.

UK patent No. 2,099,703 relates to a bone marrow biopsy needle assembly which comprises an elongate hollow needle having open front and rear ends, the front end being provided with a cutting edge: furthermore, a portion of the needle, adjacent its front end, is of a reduced internal diameter and an internal shoulder is formed between the inner end of this reduced-diameter portion and the main portion of the needle. The hollow needle is used with a trocar needle which is inserted into the hollow needle from its rear end, for a pointed front end of the trocar needle to project from the front end of the hollow needle.

In use, this assembly is gradually advanced, by hand, through the soft tissue and then through the cortical bone of the patient, by the execution of alternate clockwise and counterclockwise rotations of the assembly around its longitudinal axis. Once the assembly has advanced through the cortical bone, to reach the underlying spongy or medullary bone, the trocar needle is withdrawn from the hollow needle: then the hollow needle is advanced into the spongy bone, again by the execution of alternate clockwise and counterclockwise rotations or rotary motions of the hollow needle; it will be appreciated that a core sample of bone marrow will accordingly enter the hollow needle, from its front end. When the hollow needle has been advanced a sufficient depth into the spongy bone, about 20 to 25mm, the hollow needle is rotated several times to sever all the trabecular connections at its base and break the core sample loose from the spongy bone, and is then withdrawn. The core sample of bone marrow is retained within the hollow needle and this retention is particularly facilitated by the internal step which acts as a shoulder and also because of the fact that the core sample expands in diameter in the wider portion of the needle, inwardly or to the rear of the front end portion of reduced internal diameter. The core sample is subsequently removed from the hollow needle by inserting an elongate pusher rod into the hollow needle from its front end.

The above-described biopsy needle assembly is effective in use but it is necessary to make the hollow needle by hand, in order to provide its front end portion of reduced diameter: the hollow needle is accordingly expensive to manufacture.

WO82/03167 again discusses a biopsy needle but due to its simple structure, with no serrations distant from the end of the device, there is reduced efficacy in cutting material to obtain a biopsy sample.

In accordance with the present invention, there is provided a needle for use in taking a bone marrow biopsy, the needle comprising a hollow tube having a front end portion with a swaged reduced diameter characterised in that the outer surface of the hollow tube is formed with a series of flutes or serrations, spaced apart around its circumference, at a transition between its reduced-diameter front end portion and the main portion of the tube.

Preferably the front end of the hollow tube is formed with a cutting edge: preferably the front end of the hollow tube is tapered by means of a series of facets spaced apart around its circumference.

The flutes or serrations serve for cutting the cortical bone, by alternate clockwise and counterclockwise rotations of the hollow needle, to allow the larger-diameter main portion of the needle to advance smoothly and without resistance through the cortical bone.

It will be appreciated that the above-defined hollow needle will be used with a trocar needle to form a biopsy needle assembly for use in taking a bone marrow biopsy. This biopsy needle assembly is used in the same manner as the assembly of the above-noted UK patent No. 2,099,703. However, the hollow needle does not have to be made by hand: the swaging process, to form the reduced-diameter front end portion of the hollow needle, may be carried out by machine and the hollow needle is therefore less expensive to manufacture.

Also in accordance with the present invention, there is provided a method of forming a needle for use in taking a bone marrow biopsy, the method comprising providing a hollow tube and swaging a front end portion of the tube to provide a front end portion with a reduced diameter, with the outer surface of said hollow tube also being provided with a series of flutes or serrations, spaced apart around its circumference, at a transition between its reduced-diameter front end portion and the main portion of the tube.

An embodiment of the present invention will be described by way of example only and with reference to the accompanying drawings, in which:

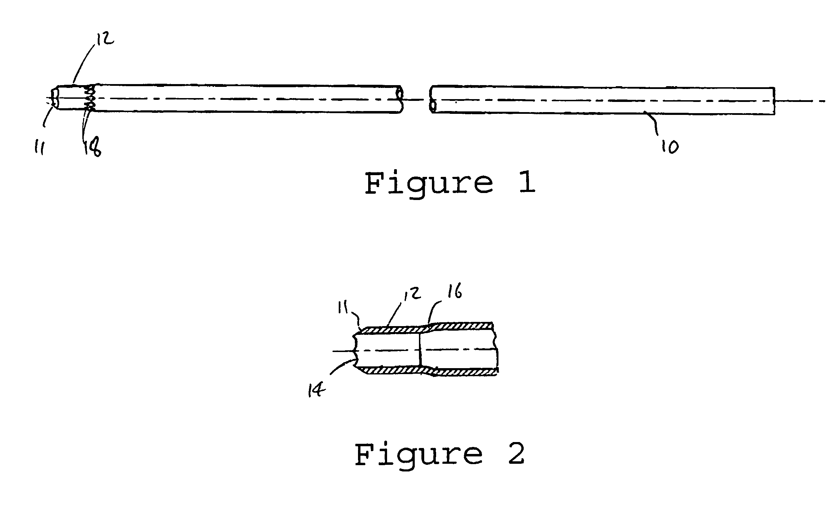

FIGURE 1 is a side view of a hollow metal needle in accordance with the present invention, for use in a bone marrow biopsy needle assembly, the hollow needle being shown without its handle and without the trocar of the assembly;
FIGURE 2 is an enlarged longitudinal sectional view of the needle adjacent its front end; and
FIGURE 3 is a view showing use of the hollow needle when taking a bone marrow biopsy.

Referring to the drawings, there is shown a hollow needle for use in a disposable bone marrow biopsy needle assembly: the hollow needle comprises a stainless steel tube 10 of circular cross-section and uniform diameter a front end portion 12 of which has been formed to a reduced diameter by swaging. The front end of the tube 10 is tapered by means of six equi-angularly spaced facets 11, thus forming a cutting edge 14. The swaging process, for forming the front end portion to its reduced diameter, results in the formation of a generally frusto-conical transition portion 16 between the main portion of the tube 10 and the front end portion 12: the outer surface of this transition portion 16 is formed with twelve equi-angularly spaced flutes 18.

The biopsy needle assembly further comprises a trocar needle (not shown) for insertion into the hollow needle 10 from its rear end, such that the pointed end of the trocar needle projects from the front end of the hollow needle, in the same manner as the assembly of UK patent No. 2,099,703: the hollow needle has a transverse handle attached to it at its end and the trocar has a domed handle attached to it at its rear end.

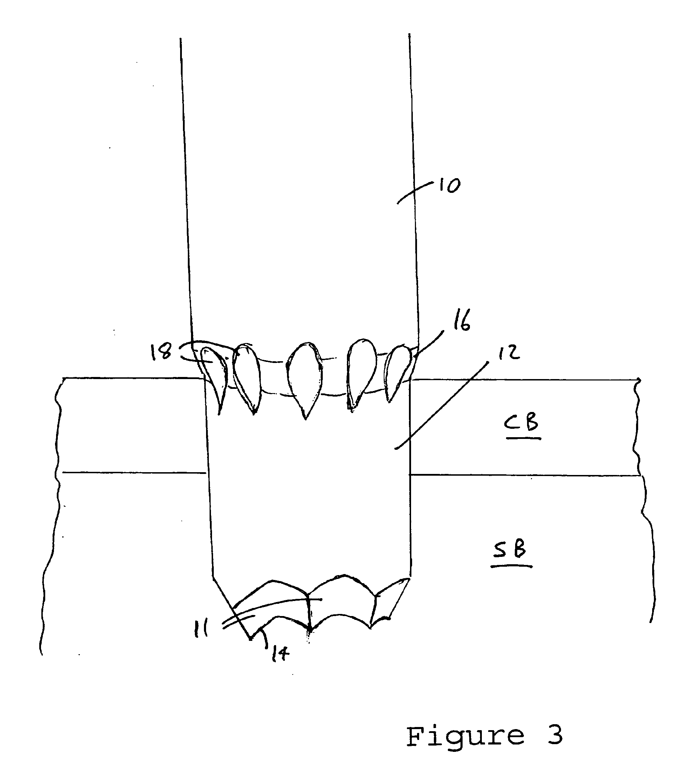

The biopsy needle assembly of the present invention is used in the same manner as the assembly of the above-noted UK patent. When the assembly reaches the spongy bone SB (Figure 3), the trocar needle is withdrawn. The hollow needle 10 is then advanced further, the flutes 18 on the transitional portion 16 serving to cut through the cortical bone CB, as the needle is turned alternately clockwise and anticlockwise, to allow the larger-diameter main portion of the hollow needle to pass through the cortical bone. When the hollow needle 10 has been advanced a sufficient depth into the spongy bone, the hollow needle is rotated several times to cut all the trabecular connections and break the core sample loose from its base, and is then withdrawn. Using this device, the core sample is retained within the hollow needle, this retention being facilitated by the internal step which acts as a shoulder and also because of the fact that the core sample expands in diameter in the wider internal portion of the needle, inwardly or to the rear of the transition portion 16.

In contrast to the biopsy needle of UK patent No. 2,099,703, the hollow needle 10 does not require to be made by hand: in particular, the swaging process, to form the reduced-diameter front end portion 12 may be carried out by machine; also, the tapering facets 11, to form the cutting edge 14, and the flutes 18 may all be formed by machine. The biopsy needle is accordingly relatively easy to manufacture, less expensive and more importantly enabling a large number of them to be produced in a short time span, which is essential where the needles are intended for single-use.

## Claims

1. A needle for use in taking a bone marrow biopsy, the needle comprising a hollow tube (10) having a front end portion (12) with a swaged reduced diameter, **characterised in that** the outer surface of said hollow tube (10) has a series of flutes (18), spaced apart around its circumference, at a transition between its said reduced-diameter front end portion (12) and the adjacent portion of the hollow tube (10).

2. A needle as claimed in claim 1, in which said front end of said hollow tube (10) is formed with a cutting edge (14).

3. A needle as claimed in claim 2, in which said front end of said hollow tube is tapered by means of a series of facets (11) spaced apart around its circumference.

4. A biopsy needle assembly which comprises a needle as claimed in any preceding claim, together with a trocar needle for insertion into said hollow tube (10) from its rear end such that the pointed front end of the trocar needle projects from the front end of said hollow tube.

5. A method of forming a needle for use in taking a bone marrow biopsy, the method comprising providing a hollow tube (10) and swaging a front end portion (12) of the tube to provide a front end portion (12) with a reduced diameter, with the outer surface of said hollow tube also being provided with a series of flutes (18) or serrations, spaced apart around its circumference, at a transition between its reduced-diameter front end portion and the main portion of the tube.

## Patentansprüche

1. Nadel zur Verwendung in der Entnahme einer Knochenmarksbiopsie, wobei die Nadel eine hohle Röhre (10) mit einem vorderen Endabschnitt (12) mit einem eingesenkten, reduzierten Durchmesser umfasst, **dadurch gekennzeichnet, dass**
die äußere Oberfläche der hohlen Röhre (10) eine Serie von Auskehlungen (18) hat, die um ihren Kreisumfang herum an einem Übergang zwischen ihrem im Durchmesser reduzierten vorderen Endabschnitt (12) und dem anschließenden Abschnitt der hohlen Röhre (10) versetzt angeordnet sind.

2. Nadel nach Anspruch 1, in der das vordere Ende der hohlen Röhre (10) mit einer Schneidkante (14) ausgeformt ist.

3. Nadel nach Anspruch 2, in der das vordere Ende der hohlen Röhre durch eine Serie von Facetten (11) abgeschrägt ist, die um ihren Kreisumfang herum versetzt angeordnet sind.

4. Anordnung von Biopsienadeln, die eine Nadel nach jedem der vorherigen Ansprüche zusammen mit einer Trokarnadel zur Insertion in die hohle Röhre (10) von ihrem hinteren Ende her umfasst, so dass das spitz zulaufende Frontende der Trokarnadel aus dem Frontende der hohlen Röhre herausragt.

5. Verfahren zur Formung einer Nadel zur Verwendung in der Entnahme einer Knochenmarksbiopsie, wobei das Verfahren das Bereitstellen einer hohlen Röhre (10) und das Einsenken eines vorderen Endabschnitts (12) der Röhre umfasst, um einen vorderen Endabschnitt (12) mit reduziertem Durchmesser bereitzustellen, wobei die äußere Oberfläche der hohlen Röhre auch mit einer Serie von Auskehlungen (18) oder Kerben bereitgestellt wird, die um ihren Kreisumfang herum an einem Übergang zwischen ihrem vorderen Endabschnitt mit reduziertem Durchmesser und dem Hauptabschnitt der Röhre versetzt angeordnet sind.

## Revendications

1. Aiguille pour l'utilisation dans une biopsie de moelle osseuse, l'aiguille comprenant un tube creux (10) ayant une partie d'extrémité avant (12) comportant un diamètre réduit estampé, **caractérisée en ce que** la surface externe dudit tube creux (10) a une série de rainures (18) espacées les unes des autres sur son pourtour, au niveau d'une transition entre ladite partie d'extrémité avant (12) à diamètre réduit et la partie adjacente du tube creux (10).

2. Aiguille selon la revendication 1, dans laquelle ladite extrémité avant dudit tube creux (10) est formée d'une arête coupante (14).

3. Aiguille selon la revendication 2, dans laquelle ladite extrémité avant dudit tube creux est effilée par une série de facettes (11) espacées les unes des autres autour de son pourtour.

4. Ensemble d'aiguille de biopsie qui comprend une aiguille selon l'une quelconque des revendications précédentes, conjointement avec une aiguille de trocart pour l'insertion dans ledit tube creux (10) à partir de son extrémité arrière de sorte que l'extrémité avant pointue de l'aiguille de trocart se projette de l'extrémité avant dudit tube creux.

5. Procédé de formation d'une aiguille pour l'utilisation dans une biopsie de moelle osseuse, le procédé comprenant la fourniture d'un tube creux (10) et l'estampage d'une partie d'extrémité avant (12) du tube pour donner une partie d'extrémité avant (12) présentant un diamètre réduit, la surface externe dudit tube creux étant également dotée d'une série de rainures (18) ou de dentelures, espacées les unes des autres sur son pourtour, au niveau d'une transition entre sa partie terminale avant à diamètre réduit et la partie principale du tube.
